Europäisches Patentamt

European Patent Office

Office européen des brevets

(11) Publication number: **0 178 790 B1**

# (12) EUROPEAN PATENT SPECIFICATION

(45) Date of publication of patent specification: **18.09.91**  (51) Int. Cl.⁵: **G01N 33/53**, G01N 33/52, G01N 33/58

(21) Application number: **85306572.0**

(22) Date of filing: **16.09.85**

(54) Processes and materials for carrying out specific binding assays.

(30) Priority: **14.09.84 GB 8423227**

(43) Date of publication of application:
**23.04.86 Bulletin 86/17**

(45) Publication of the grant of the patent:
**18.09.91 Bulletin 91/38**

(84) Designated Contracting States:
**AT BE CH DE FR GB IT LI NL SE**

(56) References cited:
**EP-A- 0 017 908**
**EP-A- 0 075 379**
**GB-A- 2 019 562**

**CLINICAL CHEMISTRY, vol.29, no.8, August
1983, Washington, DC (US); I.WEEKS et al.,
pp.1474-1479**

**CHEMICAL ABSTRACTS, vol.96, no.21, 24
May 1982, Columbus, OH (US); p.376,
no.177453h**

**CHEMICAL ABSTRACTS, vol.94, no.3, 19 Jan-
uary 1981, Columbus, OH (US); K.ZAITSU et
al., p.125, no.12014m**

(73) Proprietor: **UNILEVER PLC**
**Unilever House Blackfriars P.O. Box 68
London EC4P 4BQ(GB)**

(84) Designated Contracting States:
**GB**

Proprietor: **UNILEVER NV**
**Burgemeester s'Jacobplein 1 P.O. Box 760
NL-3000 DK Rotterdam(NL)**

(84) Designated Contracting States:
**BE CH DE FR IT LI NL SE AT**

(72) Inventor: **Davis, Paul James**
**The Hawtorns Pavenham Road
Felmersham Bedfordshire MK43 7EX(GB)**
Inventor: **Bosley, John Anthony**
**10 Philip Way Higham Ferrers
Wellingborough Northamptonshire NN9
8LG(GB)**

(74) Representative: **Butler, David John et al**
**Unilever PLC Patents Division P.O. Box 68
Unilever House
London EC4P 4BQ(GB)**

## Description

This invention relates to improvements in processes and materials for carrying out specific binding assays.

Included among specific binding assays are the well-known immunoassays, as well as corresponding assay schemes in which, instead of an immunological pair, e.g., antigen or hapten and antibody, another kind of specific binding pair is involved, such as a hormone and its corresponding binding protein from serum, or biotin and avidin or streptavidin.

A variety of techniques is known for carrying out enzyme-linked and other forms of label-linked immunoassay, in which an enzyme or other label fixed to a member of a specific binding pair involved in the assay, has its activity determined as an index of the amount of labelled material present in the free or bound state. The prior art is rich in variety of patterns of reaction scheme and assay configuration, as well as in the variety of particular specific binding pairs involved in the assays. Illustrative examples of specifications describing such assays are: USP 3 654 090, USP 3 791 932, USP 4 376 110, GB 2 074 727, EP 0 014 530, and EP 0 042 755.

Also included in the prior art are devices for carrying out immunotests for antigens, in which reagent zones are arranged in the form of solid or fibrous layers containing various reagents needed for the tests (GB 2 111 676, (Liotta)). In the devices of GB 2 111 676, a layer of immobilised antigen intervenes between a layer where sample and enzyme-linked antibody are applied, and a colour-forming layer, so that in principle only the labelled antibodies bound to the sample can penetrate to the colour-forming layer.

Specification GB A 2 023 816 (Damon) describes immunotests in which an antibody for carrying out an immunotest of the separation type is occluded within semipermeable nylon microcapsules: the test is specific for a freely diffusible analyte (such as thyroxin or cortisol etc) and the free or bound portion after a binding reaction is estimated from the quantity of labelled material within or outside the capsules.

The prior art also contains many other clinical assay arrangements, e.g. glucose indicators as exampled in GB 2 036 963 (Miles) and GB 2 026 160 (VEB Arzneimittelwerk Dresden), EP 0 058 334 (Miles) and EP 0 078 971 (Behringwerke), and the devices and tests of EP 0 110 173 (Lifescan), WO 84/02004 (Quidel) and WO 79/01081 (Battelle).

USP 4 233 402 (Syva) discloses so-called "enzyme channelling" immunoassays, which involve conjugates of two different enzyme labels, one with each of two complementary specific binding partners. The product of the first enzyme (e.g., hydrogen peroxide from glucose oxidase) is a substrate for the second enzyme (e.g. horseradish peroxidase), and the molecular coupling between the two different enzyme molecules in the resulting immune complexes is mentioned as giving enhanced production of colour product. Further enzyme channelling assays are mentioned in "Monoclonal Antibodies and Developments in Immunoassay" (1981, Elsevier) pp 109-119, in an article entitled "Homogeneous Enzyme Immunoassay Techniques for Proteins", by E.F. Ullmann, in one example of which the conjugate of one enzyme and binding pair member is coupled to agarose beads, and catalase is supplied to consume hydrogen peroxide which escapes without reacting with bound enzyme, to minimise its reaction with unbound enzyme. GB 2019562 (Syva) describes such systems in detail.

The present invention aims to provide a number of novel assay arrangements which can be widely applied to many different analytes, with a common reaction pattern which provides relative simplicity and convenience in use.

According to the invention there is provided a process for carrying out a label-linked specific binding assay, e.g. an immunoassay, comprising the steps of

- providing in an aqueous reaction zone a member of a specific binding pair to participate in the assay, linked to a label which either is a first coreagent which is reactive with a second coreagent in a reaction to produce a detectable assay result, or is a generator of said coreagent;
- providing a dispersed quantity of said second coreagent, or of a generator of said second coreagent, occluded or entrapped within a discretely bounded zone during production of the bounded zone, the bounded zone comprising cross-linked polymer, a polymer membrane microcapsule or a liposome, adjacent to said aqueous reaction zone, which bounded zone also carries in immobilised form at the zone boundary a member of a specific binding pair;
- providing in said aqueous reaction zone a consumer of one of said coreagents in said reaction, thereby to ensure the substantial absence of said coreagent in said aqueous reaction zone;
- wherein said discretely bounded zone comprises a barrier sufficient to prevent entry of the consumer reagent from said aqueous reaction zone into the bounded zone, thereby to separate the consumer reagent from the dispersed quantity of second coreagent or generator thereof within said bounded

2

zone; and

- allowing the specific binding reactions to take place whereby the assay reaction results in the binding of a variable quantity, dependent on the quantity of analyte present in the assay system, if any, of said labelled member of said specific binding pair directly or indirectly to said immobilised specific binding material, so that said detectable assay result is produced by reaction of bound label or said coreagent generated by said bound label with said coreagent occluded or entrapped within, or generated within, said bounded zone.

Also provided by the invention is a test kit for carrying out the said specific binding assay, e.g. an immunoassay, the test kit comprising

- a member of a specific binding pair that participates in the binding reaction of interest in the assay, linked to a label material which either is a first coreagent which is reactive with a second coreagent in a reaction to produce a detectable assay result, or is a generator of said first coreagent,
- a bounded disperse reagent preparation, comprising cross-linked polymer particles, polymer membrane microcapsules or liposomes, which constitutes a discretely-bounded zone containing occluded or entrapped therein a dispersed quantity either of said second coreagent in said reaction, or of a generator of said second coreagent, said bounded disperse reagent preparation also carrying in immobilised form at the zone boundary a member of a specific binding pair, and
- a consumer reagent capable of consuming one of said coreagents,
- wherein the zone boundary provides a barrier sufficient to prevent entry of the consumer reagent into said discretely-bounded zone, thereby to separate the consumer reagent from the dispersed quantity of second coreagent or generator thereof in said zone,
- wherein the bounded disperse reagent preparation and the labelled material and the consumer reagent are arranged so that they can be put into contact during performance of the assay, with the labelled material and the consumer reagent dispersed in an aqueous zone adjacent to the bounded disperse reagent preparation, whereby in use the consumer reagent ensures the substantial absence of one of said coreagents in said aqueous reaction zone, and the assay reaction results in the binding of a variable quantity of said labelled material directly or indirectly to said immobilised binding material, so that said detectable assay result is produced by reaction of said coreagent in said bounded disperse reagent preparation with said label bound thereto or with coreagent generated by said label.

Several examples of the invention correspond to an assay system (and corresponding process and test kit) for carrying out a label-linked specific binding assay (e.g. an immunoassay), wherein a label is used which either is a first coreagent which is reactive with a second coreagent in a reaction to produce a detectable result, or is a generator of said first coreagent, said label being linked to a member of a specific binding pair relevant to the assay, to form a labelled specific binding material, said assay system comprising a bounded zone containing a dispersed quantity of said second coreagent in said reaction, or a generator of said second coreagent, said bounded zone also carrying an immobilised member of said specific binding pair, an aqueous reaction zone comprising a consumer of one of the coreagents in said reaction, together with said labelled specific binding material, whereby in use the assay reaction results in the binding of a variable quantity of said labelled member of said specific binding pair in said bounded zone, so that said detectable result is produced by reaction of the coreagent in the bounded zone with the labelling material or the coreagent generated by said labelling material.

In the assay system, the first coreagent can be for example a participant in a luminescent reaction, or an enzyme that catalyses directly or indirectly a luminescent or chromogenic or fluorogenic reaction, and the mentioned second coreagent is for example a further reactant in said reaction or an enzyme that catalyses said reaction.

A generator of said coreagent can be for example an enzyme that catalyses a reaction of which the product is said coreagent, or a system that generates said co-reagent non-enzymically.

The bounded zone can be for example a discrete zone, for example that formed by a gel or capsule or bead, and the surface thereof, optionally together with the zone molecularly adjacent to said surface, i.e., that zone which contains any molecules directly or indirectly sorbed to said surface.

The immobilised member of said specific binding pair can for example be carried on said surface, and can be fixed there by per se known technique for immobilising specific binding materials.

The said second coreagent in said reaction (or generator thereof) which is dispersed in said bounded zone is occluded or entrapped there, e.g. by occlusion of free coreagent or generator within liposomes or membrane microcapsules or entrapment during polymerisation of a polymer such as acrylic polymer.

The boundary between the bounded and the aqueous reaction zones in examples described below is such as to prevent the consumer reagent (which can be for example an enzyme) from penetrating or

3

dispersing or diffusing into said bounded zone, and in many cases also prevents the labelled specific binding material from penetrating further than the surface and/or the molecularly adjacent layer of the bounded zone.

The discretely-bounded zone can contain either an enzymic or non-enzymic generator of the second coreagent, or a supply of the second coreagent.

In particular examples of the test kit, a first reagent preparation comprises a preparation of beads or capsules to provide the bounded zone, carrying immobilised specific binding material and containing a hydrogen-peroxide-generating system as second coreagent-generating system, and said second reagent preparation comprises a second specific binding material, complimentary to the first, linked to a peroxidase enzyme or a peroxide-reactive chemiluminescent label material, the text kit also comprising catalase (in use to consume any hydrogen peroxide in the aqueous reaction zone outside the beads or capsules), and optionally also a chromogenic or fluorogenic or luminescent material to give a detectable result upon reaction with the label and the reactant.

Usually, the label is brought into contact with the second coreagent in the bounded zone by arranging that there is a determinate quantity of specific binding material located in immobilised form in the bounded zone, to bind a variable quantity of the label linked to one of the specific binding partners in the assay reaction, according to the quantity of analyte present in the test system.

According to a convenient arrangement within the scope of the invention, the second coreagent is diffusible, is supplied or provided in the bounded zone, and its effective absence from the aqueous reaction zone is assured by a consumer reagent in the aqueous reaction zone that effectively consumes the coreagent diffused from the bounded zone, i.e. to prevent it from undergoing appreciable or substantial reaction with the label in the aqueous reaction zone.

The label and the second coreagent are chosen so that they can react together in the bounded zone to produce a detectable result. In many examples, a suitable label is for example an enzyme, and a detectable product results from reaction of the enzyme and the second coreagent, e.g. a coloured or fluorescent product, or a result that is detectable in some other way, e.g. as a luminescent reaction. In many examples of this system, a co-reactant is present, which reacts with the second coreagent and enzyme to produce the product. (The second coreagent can be for example a peroxide such as hydrogen peroxide, the co-reactant can be a chromogen or fluorogen or a reactant in a luminescent reaction, and the enzyme can be a peroxidase.) Any co-reactant in the system can (but need not) be localised to the bounded zone: it can for example be soluble and present in solution in aqueous liquid forming the aqueous reaction zone.

An alternative suitable enzyme, to be occluded within gel or microcapsules, is yeast methanol oxidase, supplied with substrate quantities of methanol and oxygen, thereby to supply hydrogen peroxide as a reactant for the assay.

In alternative embodiments, the label need not be an enzyme: it can for example be a luminescent substance. A suitable example of such a substance is an N-methylated product of a 9-acridine-carboxyl group, which reacts with $H_2O_2$ as the reactant, to give a light output. Where appropriate, references to "enzyme" herein include the case of another type of reactive label.

These arrangements can for example be provided by means of a biphasic or two-compartment system in which diffusible coreagent is supplied or provided, e.g. by a coreagent-generating system, in the first phase or compartment and diffuses into the second phase or compartment where it is consumed by the consumer reagent.

This arrangement offers the advantage of example of enabling the production of immunoassays in which the result is indicated by a colour reaction, with unusually definite endpoints at high or low analyte levels, and very few liquid addition operations. Often the minimum rate of colour formation attainable in a chromogenic immunoassay is an appreciable fraction of the maximum rate of colour formation, and many liquid addition operations are needed.

In a further alternative embodiment, the bounded zone of the reaction system can contain a chromogenic, fluorogenic or luminescent coreagent or plural number of coreagents, e.g., horseradish peroxidase with one of its substrates mentioned herein, carried in for example a polymer gel which is sufficiently crosslinked to exclude entry of catalase, and carrying specific binding activity fixed to the gel surface, and the label can be a generator of a remaining coreagent, e.g. glucose oxidase to generate hydrogen peroxide. In this arrangement the unbound labelled material generates hydrogen peroxide coreagent in the aqueous liquid adjacent to the gel, but the consumer reagent (catalase) consumes it. Upon binding of the labelled material to the gel surface the generated coreagent can produce a detectable result by reaction with the materials contained within the gel.

The provision of a consumer reagent or otherwise of a reactant-free zone enables the minimum rate of colour formation to be brought down much lower than usual and ideally to zero.

4

A further advantage of this assay arrangement lies in the facility to provide all reagents together in one reaction volume. There is no need to separate free labelled material from bound labelled material, nor to separate the reaction liquid for the specific binding step(s) from the reaction liquid in which a chromogenic or fluorogenic enzyme reaction takes place in order to enable detection of the assay result.

Using the techniques described herein, it is possible and advantageous to add in a single reaction liquid or reaction system all the components of the assay including the chromogen (or fluorogen or other co-reactant such as luminescent material).

The bounded zone of the assay system of the invention can effectively comprise the first phase or compartment of the biphasic or two-compartment system mentioned above, together with so much of the interphase or interfacial region as contains appreciable supplies of the reactant, and the aqueous reaction zone effectively comprises the rest of the second phase or compartment.

Under these conditions, the enzyme can be brought into contact with the second coreagent by a binding event which fixes it in the interphase or interfacial zone. For example, the interfacial or interphase zone can include a gel surface or a membrane having fixed thereto specific binding sites to which the enzyme can become bound, e.g. in the form of a conjugate with a member of the specific binding pair relevant to the assay, to an extent dependent on the quantity (if any) of analyte (which can be one member of the specific binding pair) present in the system. The specific binding sites can be provided by (for example) per se conventional techniques for fabrication of immunoadsorbents.

The way in which the enzyme becomes bound can correspond to any of the ways in which enzymes have heretofore been bound to immunoadsorbents during the course of immunoassay. For example, in several arrangements an enzyme linked to an antigen or hapten can become bound to an antibody immunosorbent in inverse relation to the quantity of analyte antibody (or antigen or hapten) which is available for previous binding or completion with respectively the enzyme conjugate or the immunoadsorbent. This is the pattern tht occurs in for example a competitive immunoassay. Alternatively the enzyme linked to an antibody or antigen can become bound to immunosorbent in direct proportion to the quantity of analyte that can be bound both by the immunosorbent and by the conjugate although these latter do not otherwise appreciably bind with each other. This is the pattern that occurs in for example a "sandwich" immunoassay or an antiglobulin test or an immunoglobulin capture assay.

For example, a competition assay can be arranged by attachment of antibody to the interphase or interfacial zone, e.g. anti-beta(2)microglobulin or anti-C-reactive protein, and the enzyme to be brought into effective contact with the reactant can be provided in a form in which it is conjugated, by any suitable known conjugation technique, to the corresponding antigen, namely beta(2)microglobulin or C-reactive protein. The analyte in such a case can be the corresponding antigen itself.

By way of further example, a sandwich assay can be arranged again by attachment of antibody to the interphase or interfacial zone, e.g. anti-ferritin or anti-(hepatitis B surface antigen) or anti-(immunoglobulin E). A further antibody also with specificity for the same antigen can then be used in the form of its conjugate with the enzyme to be brought into effective contact with the second coreagent

It is within the scope of the invention to link the label to the specific binding material or bindable material in an indirect manner, e.g. as a label occluded in a preparation of microcapsules or liposomes (such as those described in EP 0 014 530) and/or a label linked to a further binding material (such as an antiglobulin) with affinity for the material which it is intended to label.

It will be apparent to the reader that any specificity can be imparted to the assay materials provided by this invention upon appropriate choice of specific binding components.

One convenient method of providing an interphase or interfacial zone including an immunosorbent capacity is to provide beads or capsules containing a second coreagent-generating system together with an immunosorbent surface. For example, polyacrylamide beads containing a second coreagent-generating system can be provided with immunosorbent surfaces.

In one very suitable example of a second coreagent-generating system, an enzyme to generate the coreagent is immobilised or occluded in the bounded zone, e.g. dispersed within a bead or capsule, e.g. a crosslinked polyacrylamide bead, but unable itself to escape into the aqueous reaction zone, which can be liquid surrounding the bead or capsule. A suitable example preparation of beads or capsules for this purpose comprises beads of 20-500 μm (micron) diameter, sufficient to allow free diffusion of low molecular weight coreagents, e.g. made by suspension or emulsion polymerisation of acrylamide with crosslinker.

In alternative examples, the second coreagent-generating system can be contained within liposomes bearing specific binding material on their surface, e.g. those mentioned in EP specification No. 0 014 530 and references cited therein, and examples given herein below.

A suitable enzyme to generate the second coreagent is for example glucose oxidase (provided with glucose and oxygen), which enzyme can be for example occluded within a polyacrylamide bead which has a degree of crosslinking sufficient to prevent any appreciable diffusion of the glucose oxidase out of the bead. The occluded glucose oxidase enzyme can be provided with its substrates glucose and dissolved oxygen by for example diffusion from outside the bounded zone, e.g. by diffusion from liquid surrounding the bead or capsule. This system generates hydrogen peroxide ($H_2O_2$) within the bead, and the $H_2O_2$ diffuses out freely from the bead into the surrounding liquid, where it can be used as the second coreagent. This example of a coreagent for the rest of the assay system ($H_2O_2$) can alternatively be produced by any other convenient generating source in the bounded zone, and diffuses through the interphase or interfacial zone into the aqueous reaction zone, e.g. surrounding liquid, where the consumer reagent can for example be a suitably chosen concentration of catalase enzyme.

An alternative analogous second coreagent is for example glucose-6-phosphate, which can be released for example from a gel preparation (as the bounded zone) which occludes a sufficient supply of glucose-6-phosphate, e.g. in solid slowly-dissolving form. For this purpose the consumer reagent present outside the bounded zone can be glucose-6-phosphatase, and a corresponding enzyme label is glucose-6-phosphate dehydrogenase. In this case the coenzyme NADP is supplied, and the extent of its reduction can be detected in the usual (e.g. spectrophotometric or fluorometric) manner, and is directly related to the amount of the enzyme which has become bound to the gel surface.

The glucose-6-phosphate can be provided for example in the form of a finely-divided solid carried within a hydratable polymer gel, e.g., an acrylic polymer gel which is sufficiently crosslinked to exclude the consumer reagent, e.g. such a gel which has been produced by polymerisation of an acrylic comonomer mixture carrying the finely-divided solid particles in suspension, in non-aqueous solvent.

Conversion of such beads to specific binding material of desired specificity can be achieved in any suitable way, but in particular by incorporating biotin, e.g. in the manner described below, or a hapten, and then absorbing the biotin or hapten with e.g. avidin or anti-hapten conjugated to an antibody, of desired specificity.

Alternative second coreagent-generating systems not involving enzymes can also be chosen. For example, where hydrogen peroxide is the coreagent which it is desired to generate, the generating system can be a chemical hydrogenperoxide generating system, e.g. a photochemical system, in which the bounded zone is illuminated with light which is actinic for material which can reduce oxygen under photo activation in the presence of an electron-donor, e.g. flavin mononucleotide (FMN), flavin adenine dinucleotide (FAD), anthraquinone, methyl viologen or 1,1'-ethylene-2,2'-bipyridylium in the presence of an aliphatic tertiary diamine, such as a substituted ethylene diamine, e.g. EDTA. These photochemical hydrogen-peroxide-generating systems include systems known per se. It can be useful to add superoxide dismutase enzyme in these systems to lower the concentration of photochemically-produced superoxide which might affect some of the other reagents in the system.

Preferred embodiments of the invention are described by way of example, together with details for the preparation or procurement of the principal assay materials, as follows:

Example 1

1. Preparation of principal assay materials:

A polymerisable biotin derivative, the 2-hydroxyethyl methacrylate ester of biotin, can be prepared by the following process:-

(a) Add 10ml of thionyl chloride to 1g of d-biotin (from Sigma Chemical Co. (Trade Mark)), keeping the temperature in the range 0-5°C. Leave for 1 hour with occasional shaking. Filter off the precipitate formed, and separate any unprecipitated product from the filtrate by adding 20ml of a 1:1 toluene-petroleum ether (100-120°) mixture. Wash the combined precipitates with a further 20ml of the solvent mixture.

(b) Use the biotinyl chloride product for the next stage immediately. Store any unused product over $P_2O_5$ after vacuum oven drying at 25°C.

Dissolve 0.5g of the biotinyl chloride in a mixture of 42.5g of 2-hydroxyethylmethacrylate (HEMA) and 0.5g pyridine. Adjust the pH to approximately 7 by the addition of more pyridine. Stir at 20°C for two hours. Add the mixture to 400ml toluene, and then add 400 ml water. Shake, and then remove the organic layer. Add a further 400 ml water and repeat the extraction. Dry the organic layer over anhydrous magnesium sulphate. Remove toluene on a rotary evaporator, keeping the temperature less than 60°C. Store the product at 0-5°C. It contains the 2-hydroxyethylmethacrylate ester of biotin (biotin-HEMA ester) monomer in admixture with a substantial amount of HEMA.

(c) The following description shows use of the biotin-HEMA monomer prepared as described in parts (a) and (b) above to prepare copolymers with acrylamide and methylenebisacrylamide in bead form containing (occluded) active glucose oxidase enzyme, and having covalently-attached biotin groups at the surface, derived from the polymerisable biotin derivative comonomer. The polymer is sufficiently cross-linked to prevent avidin-enzyme (HRPO) conjugates from entering by penetrative diffusion, and to prevent the occluded enzyme from diffusing out, when the beads are suspended in aqueous liquid.

(d) Dissolve 7g acrylamide and 0.48g methylenebisacrylamide (MBA) in 32ml of sodium acetate buffer (SAB, pH 5.6) and then add 0.6g of the product from the biotin-HEMA monomer preparation (parts (a) and (b) above). (SAB contains 2.84ml acetic acid (glacial) + 99ml water, and is adjusted in pH to 5.6 with sodium hydroxide solution, and diluted to 1 litre with water).

Deoxygenate by bubbling nitrogen through the mixture for fifteen minutes. Then add 3ml of an ammonium persulphate solution (0.4g/ml) and 8ml of a glucose oxidase solution (2mg/ml, in SAB). Pour the mixture into a 500ml glass bowl containing 350ml n-hexane and 0.9g SPAN 80 detergent (Trade Mark).

Stir the mixture with an overhead stirrer fitted with a "U" shaped glass stirrer contoured to the bottom and sides of the bowl. Adjust the speed to approximately 220 rpm. Stir for one minute after the addition of the monomer mixture. Then increase the speed to about 250 rpm. Add 2.2ml tetramethylethylenediamine (TEMED) and continue stirring for three minutes. Turn the speed back down to 220 rpm and continue for one hour. (The bowl should be covered during stirring to reduce evaporation of hexane).

Decant off most of the hexane, filter off the beads, and wash with a large volume of SAB. Remove any large lumps of polymer, and store in SAB with a small amount of thimerosal (antimicrobial preservative) at 0-5° C.

Examples of beads prepared according to the above procedure have been found to have a particle size range of about 30-500 microns and to show swelling (in SAB) of 5.1g/g and glucose oxidase activity equivalent to about $1.3 \times 10^{-4}$g (enzyme)/g (dry polymer).

(e) An alternative polymer bead preparation, similar to that described in part (d) but containing a lower proportion of biotin groups, can be made as follows. The procedure is the same as described in part (a) above, except for the following modifications. The monomer mixture is made with 1ml of a solution of the biotin-HEMA monomer product in water ($6.6 \times 10^{-3}$g/ml + 7g acrylamide + 0.48g MBA + 32ml SAB. Examples of beads prepared according to this modified procedure have shown a swelling in SAB of 5.3g/g and a glucose oxidase activity equivalent to about $1.1 \times 10^{-4}$g (enzyme)/g (dry polymer).

The following details refer to an assay for biotin, using among other materials avidin-peroxidase conjugate. It will, however, be clear to the skilled reader that the polymer beads prepared as described above can be converted to form an immunoadsorbent of desired specificity by first adsorbing them with a conjugate of avidin or streptavidin linked in known manner (e.g. by use of a known glutaraldehyde coupling technique) to an appropriate antigen or antibody or specific binding analogue or specific binding competitor of an antigen or antibody of appropriate specificity.

Then the desired form of immunoassay or other specific binding assay can be arranged by using the peroxidase conjugate of an appropriate specific binding agent in place of the avidin-peroxidase conjugate used as described below. The format of the immunoassay can be chosen from among any of those described hereinabove, or any other suitable assay format.

2. Performance of assay:

Materials for the performance of an example specific binding assay include:

(a) polymer beads with covalently attached biotin and occluded glucose oxidase, as prepared in parts 1-(a)-(d) above;

(b) a conjugate of horseradish peroxidase (HRPO) with avidin D, either as obtained commercially (Sera-Lab or Vector Laboratories (Trade Marks), protein concentration 5-10 mg/ml, ratio HRPO:avidin 1.5:1 to 2:1) or as prepared for example by the known glutaraldehyde coupling technique from HRPO and avidin;

(c) catalase (bovine liver catalase, thymol-free, from Sigma Chemical Co. (Trade Mark));

(d) D-glucose;

(e) 2,2'- azinodi-(3-ethylbenzthiazolinosulphonic acid) diammonium salt (from Sigma Chemical Co. (Trade Mark)) (ABTS or ADBS);

(f) a liquid sample containing an unknown quantity of biotin, diluted if need be to supply of the order of 0.001-0.1μg biotin per ml of final reaction liquid. (The precise useful assay range is calibrated in the normal way against standards, for each particular manner of preparation of the assay reagents).

Component (b), the conjugate, is used in diluted admixture with an inert protein, (ratio 200-400μg conjugate to 100mg protein), in this case bovine serum albumin, in order to stabilise it in the usual stock solutions and containers.

7

An assay of biotin using these materials can be performed either in two stages or in one stage. To perform the assay in two stages, a first stage mixture can be made, comprising the following constituents (per ml of reaction liquid):(i) 50mg of bead component (a); (ii) a standard aliquot of conjugate (b) in a quantity in the range about 7.5-15$\mu$g which is the same for all samples, calibration standards and controls in a given assay run; (iii) 3.75mg bovine serum albumin, (diluent/stabiliser, inert protein used in admixture with the stock solutions and preparations of conjugate (b)). The liquid in which stock solutions are made up for suspending and dissolving these components is phosphate-buffered saline (PBS) (containing (per litre) 8.5g NaCl, 1.07g disodium phosphate and 0.39g monosodium phosphate in purified water); and (iv) sample liquid adjusted if possible (e.g. by serial dilution) to contain about 0.003-0.3$\mu$g biotin.

The first stage mixture can be shaken for about 40 minutes to keep the polymer beads in suspension. Then the beads are separated, e.g. by centrifugation, and added (sample by sample) to respective aliquots of further reagents to make a second stage mixture containing (per 2.5ml); 50mg of treated beads from first stage mixture, 0.46mg catalase, 2mg ABTS, 10mg glucose, all in PBS solution. After 30 minutes' incubation an aliquot of supernatant liquid is taken for absorbance measurement at 630nm against a comparison blank of PBS solution containing only the ABTS, catalase, and glucose as used in the second stage reaction mixture.

The assay routine has been found to permit satisfactory assay of biotin at the levels indicated.

In alternative assays, all the reagents are mixed together in a single reaction liquid (the weights of reagents given above for the second stage can be scaled down to leave the same final concentrations). This enables a particularly convenient form of one-stage assay which can be presented by means of appropriate combinations of stock reagents so as to minimise the number of manipulations required on the part of the user.

Such an assay can be carried out for example as follows. In the following example procedure the binding reaction partners are allowed to react together before the addition of substrate and consumer enzyme (catalase), but experiments have shown that this delayed addition is unnecessary: the substrate and the enzyme can be added at the start.

Stock solutions of avidin-HRP conjugate (obtainable from Vector Laboratories (Trade Mark)) are prepared as follows:

(a) 7.5 $\mu$l avidin-HRP conjugate + 18.8 mg bovine serum albumin (BSA);
(b) 7.5 $\mu$l avidin-HRP conjugate + 18.8 mg BSA, + 7.5 $\mu$l biotin solution;
(c) 7.5$\mu$l av-HRP conjugate + 18.8 mg BSA + 75 $\mu$l biotin solution;
(d) 7.5 $\mu$l av-HRP conjugate + 18.8 mg BSA, + 150 $\mu$l biotin solution;
(e) 7.5 $\mu$l av-HRP conjugate + 18.8 mg BSA, + 225 $\mu$l biotin solution;
(f) 7.5 $\mu$l av-HRP conjugate + 18.8 mg BSA, + 375 $\mu$l biotin solution;
(g) 7.5 $\mu$l av-HRP conjugate + 18.8 mg BSA, + 563 $\mu$l biotin solution;
(h) 7.5 $\mu$l av-HRP conjugate + 18.8 mg BSA, + 750 $\mu$l biotin solution.

Biotin solutions were $4.2 \times 10^{-4}$ mg/ml in phosphate buffered saline (PBS). All the above stock solutions are diluted to 5 ml with PBS before use.

The biotin-substituted beads, (high biotin level) are filtered and weighed out into 24 x 50 mg lots. These are divided into eight lots of three and 1 ml of solution (a) is added to each bead sample of one group of three, 1 ml of (b) to each sample of another group of three, etc. (to give triplicate readings at each point). They are then put on a wrist shaker for 40 minutes.

To each bead sample 1 ml catalase solution (0.46 mg/ml, in PBS) is added, then 1 ml ABTS dye solution (2 mg/ml, in PBS) and then 0.5 ml glucose solution (20 mg/ml, in PBS). They are put on a wrist shaker.

Thirty minutes after the glucose addition, 200 $\mu$l of supernatant is removed (after having allowed the beads to settle). The optical densities are measured at 630 nm against a blank composed of 1 ml PBS + 1 ml catalase solution + 1 ml dye solution + 0.5 ml glucose solution.

Within the range of 0.6 - 35 nanogram/tube biotin the colour development of the assay tubes was found to vary between substantially its maximum and minimum values, inversely with the biotin concentration.

Example 2

In this example, gentamicin is assayed by a procedure in many ways analogous to the procedure given above for Example 1.

The components of an illustrative microbead assay system for the assay of gentamicin are in outline as follows (further description is given below):-

| Component | Description | Quantity (per tube) = 250 µl total) |
|---|---|---|
| Micro-beads | Polyacrylamide (cross-linked) with entrapped glucose oxidase and bearing on their surface sheep-anti-gentamicin antibodies | 12.5 mg (wet wt.) |
| HRP-gentamicin | Prepared by periodate oxidation of HRP and subsequent reaction with gentamicin | 665 ng HRP eqvt. |
| Catalase | Bovine liver, ex Sigma, (Trade Mark), thymol free, 10-25,000 units/mg | 100 µg |
| Buffer | Phosphate/citrate, pH 6.1 + Triton ® X-100 (1% v/v) | Used throughout |
| Substrate | Glucose (0.1 M) O-phenylene diamine (product is measured as O.D. at 405 nm) | 4 mg 160 µg |
| Samples | In range from 0.2-15 mg/litre | 50 µl |

The following section describes the preparation and characterisation of $H_2O_2$-generating beads for the gentamicin assay and the preparation of HRP-conjugates. The example bead types prepared in this way all contain active glucose oxidase: but they can differ in the functional groups arrayed on their surfaces. Beads produced for use with the avidin-HRP conjugate (substantially as in the preceding example) are given surface biotin groups, whilst beads intended for linking to amino groups on antibodies or antigens are provided with carboxyl groups (through the incorporation of acrylic acid as a comonomer).

The coupling of antibodies to carboxyl-containing beads can be accomplished in essentially known manner by means of the N-hydroxy succinimide/EDC (carbodiimide) reaction.

Preparation of acrylamide:sodium acrylate beads

Beads to be used for coupling vias the amino groups of the antibody are prepared using sodium acrylate comonomer to give surface carboxyl groups, (in the following modification of the procedure described above for biotin-substituted beads in connexion with Example 1).

The materials used include: Sodium carbonate (anhydrous); PBS buffer (pH 7.2); Acrylic acid (glacial); Acrylamide and N,N¹-methylenebisacrylamide (MBA), electrophoresis grade; Glucose oxidase, type VII ex Sigma; Ammonium persulphate; Hexane (fraction from petroleum); SPAN 80 (Trade Mark) surfactant; Tetramethylenethlenediamine (TEMED), electrophoresis grade; Thimerosal.

The suspension medium is prepared by dissolving SPAN 80 (0.4g) in hexane (350 ml) in a round bottomed glass reaction flask. The monomer solution is prepared by dissolving sodium carbonate (0.88g) in PBS buffer (33.3g). To this glacial acrylic acid is slowly added (1.19g) and the solution was stirred until effervescence ceases. Acrylamide (4.70g) and methylenebisacrylamide (0.93g) are added and allowed to dissolve. The solution is deoxygenated by bubbling with nitrogen for ten minutes. 1 ml glucose oxidase solution (15 mg/ml in distilled water) is dispersed in the monomer solution using the nitrogen stream to obtain good mixing. 1.5 ml ammonium persulphate solution (0.4 g/ml in distilled water) is added the nitrogen stream to thoroughly mix this in, over a 30 second period. The monomer mixture is poured into the warmed suspension medium (27-29°C) whilst stirring with a U-shaped glass stirrer at approximately 230 rpm. After 30 seconds 5.0 ml TEMED is added to the hexane and the flask is covered to minimise solvent loss through evaporation. The polymerisation is allowed to proceed for 30 minutes, then the stirrer speed turned down to approx. 120 rpm for a further 25 minutes. The stirrer is then stopped to allow the beads to settle. Most of the hexane is decanted off, and the beads are filtered and washed with PBS until free of both hexane and SPAN 80 surfactant. The beads are stored as a slurry in PBS (4°C) containing approx. 0.01% w/v thimerosal as preservative.

The resulting beads, as made in one example test run of this procedure, were found to contain glucose oxidase activity at the level of 0.140 μg enzyme/mg dry beads.

Anti-gentamicin antibodies are coupled to the micro-beads for example as follows.

Materials used include: Micro-beads bearing surface carboxyl groups (prepared as above), 5g (wet weight); N-hydroxy succinimide (NHS), 70 mg;
1-ethyl-3-(3-dimethylaminopropyl) carbodiimide (EDC) 100 mg; IgG fraction of sheep anti-gentamicin serum, 2mg; 0.1 M bicarbonate buffer (pH 9.8) containing 0.3% (v/v) ethanolamine; 0.1 M phosphate buffered saline, pH 7.4 (per litre: 85g NaCl, 1.07g sodium hydrogen phosphate and 0.39g disodium phosphate adjusted to pH); "PC" Phosphate/citrate buffer pH 6.1 (containing 7.75 g/l citric acid and 22.48 g/l disodium phosphate; and
"PCT" Phosphate/citrate buffer pH 6.1; containing + 1% v/v Triton X-100 (Trade Mark) surfactant.

The micro-beads are washed in distilled water to remove preservatives, etc. The washed microbeads (5g, wet wt.) are resuspended in 6 ml distilled water. NHS (70 mg) in 5 ml distilled water is added to the suspension so formed. EDC (100 mg) in 5 ml distilled water is then added, bringing the total volume to about 20 ml. The reaction mixture is roll-mixed for 1 hour at room temperature (activation). After 1 hour the micro-beads are washed with 3 x 15 ml distilled water. The washed, activated micro-beads are resuspended in 10 ml distilled water containing 3 mg anti-gentamicin IgG fraction. This activated micro-bead/IgG reaction mixture is roll-mixed overnight at 4°C to couple the antibody to the activated microbeads. The antibody-coupled micro-beads are then washed with 2 x 15 ml distilled water. Ethanolamine (0.3% v/v) in 15 ml 0.1 M bicarbonate buffer (pH 9.8) is then added to the antibody coupled micro-beads to neutralise (block) residual protein-coupling sites on the beads. The reaction mixture is roll-mixed for 1 hour at room temperature. After 1 hour the micro-beads are washed in 1 x 15 ml distilled water, 2 x 15 ml PBS pH 7.4, 1 x 15 ml phosphate/citrate buffer, pH 6.1 + 1% Triton, 1 x 15 ml phosphate/citrate buffer, pH 6.1 and, finally they are suspended in 15 ml of phosphate/citrate pH 6.1. The beads are then stored at 4°C in phosphate/citrate buffer, pH 6.1.

Gentamicin-HRP Conjugate is prepared by a technique adapted from the description by Boersma and Streefkerk (J. Immunol. Methods., 1979, 30, 245-255):-
Materials used include:- Horse radish peroxidase (HRP, Sigma type VI) (10 mg); Gentamicin sulphate (2.8 mg); Sodium m-periodate, 0.1 M aqueous solution (500 μl); Sodium borohydride (400 μg); Visking tubing (narrow bore); 0.1 M acetate buffer, pH 4.0; 1 M carbonate buffer, pH 9.5; 0.01 M phosphate buffered saline pH 7.4 (PBS).

HRP (10 mg) is dissolved in distilled water (2 ml). Sodium m-periodate (0.1 M aqueous 500 μl) is then added. This reaction mixture is rolled for 25 minutes at room temperature to oxidise sugar groups on the enzyme. After 25 minutes rolling, the mixture is dialysed overnight at 4°C against 0.1 M acetate buffer (pH 4.0). The dialysed mixture is then transferred to a reaction vial with gentamicin sulphate (2.8 mg) in 1M carbonate buffer (pH 9.5, 1 ml). This reaction mixture is rolled for 2 hours at room temperature (to couple gentamicin to HRP by Schiff base formation). After 2 hours rolling, sodium borohydride (400 $\mu$g) in distilled water (100 $\mu$l) is added. The reaction mixture is rolled for 2 hours at 4°C.

After 2 hours rolling the mixture is trransferred to narrow bore Visking (Trade Mark) tubing and dialysed overnight at 4°C against 0.01 M PBS, pH 7.4. Finally, the product (conjugate) is stored at 4°C in a stoppered brown glass vial (5 ml capacity).

Using the materials prepared above, a competitive assay for gentamicin can be carried out as follows. In this system antibodies are bound to the bead surface, and the labelled material used is gentamicin labelled with horse radish peroxidase (as described above). Free gentamicin from the sample is able to compete for the antibodies bound to the bead surface.

Materials used for assay include:-
Antibody-linked micro-beads 12.5 mg/tube; Gentamicin-HRP conjugate; Gentamicin sulphate solution (6 $\mu$g/ml in PCT Buffer); Catalase solution (in PCT), 2,000 $\mu$g/ml (ex Sigma, bovine liver, thymol free, 10-25,000 units per mg); D-glucose, 100 mg, and O-phenylenediamine, 8 mg, in 10 ml PC Buffer, pH 6.1; Phosphate citrate buffer, pH 6.1 (PC buffer); 0.01M phosphate buffered sealine, pH 7.4 (PBS); Phosphate citrate buffer, pH 6.1 + Triton x 100 (PCT).

A set of tubes (2 ml Sarstedt) is arranged, each containing 50 $\mu$l of antibody-linked micro-beads (= 100 ug per tube). Gentamicin standards (50 $\mu$l each) are then added to give replicate tubes with 0, 5, 10, 50, 100 and 500 ng gentamicin per tube. Conjugate solution (50 $\mu$l diluted 1:200) is then added to each tube, followed immediately by glucose/OPD substrate solution. All of the tubes are placed on a continuous mixer for 40 minutes in the dark at room temperature. After 40 minutes incubation, 125 $\mu$l aliquots are withdrawn from each tube, placed in the wells of a microtitre tray and their optical densities measured at 405 nm with a standard plate reader (Uniskan (Trade Mark)).

A calibration curve derived from this assay showed variation of colour development from substantially maximum to substantially minimum within the range 2-500 nanogram/tube gentamicin, with an inverse relation between gentamicin level and colour intensity.

A further useful example of an assay, based on a photochemical hydrogen-peroxide-generating system, is an antiglobulin test for mouse antibody raised against E.coli 0149 lipopolysaccharide. Suitable liposomes incorporating the antigen at their surface are as described in EP 0 014 530, except that the material occluded in the liposomes is a mixture of 0.01 M FMN and 0.01 M EDTA. A sandwich-type antiglobulin test can be arranged using a commercially-obtainable conjugate of horseradish peroxidase with sheep-anti-mouse immunologlobulin, by illuminating a reaction liquid sample containing all of the ingredients including the liposomes, antibody sample, antibody conjugate, catalase, and chromogenic peroxidase substate.

If desired, the reaction liquids can contain a suitable dense polymeric or polysaccharide suspending agent (e.g. Ficoll, (Trade Mark)) which can be provided in a suitably-chosen density of solution to eliminate the need to shake the bead suspensions.

Alternatively, the beads or other forms of polymer with the properties indicated therein can be provided in a form in which they are attached to or form part of the surface of a macro-solid phase carrier material such as a larger (e.g. 5mm diameter) bead, or a stick, peg or strip, e.g., a paper or cellulose or glass-fibre or synthetic plastics material.

As alternative materials to the liposomes mentioned above, polymer membranes, especially e.g. as capsules, can be used, such as for example those described in GB 1 540 461 (Damon), and modified by attachment of specific binding groups.

In the examples given above, a peroxidase enzyme is used as the enzyme label attached to a binding reaction partner, and hydrogen peroxide is used as reactant for the enzyme label together with a chromogenic co-reactant. Among such chromogenic co-reactants, besides 2,2'-azino-di(3-ethylbenz-thiazoline sulfonic acid) (ADBS), prcducing a green soluble product that absorbs light particularly strongly at 415 nm, other chromogens can be used, for example o-dianisidine, giving a sparingly soluble yellow-orange product that absorbs especially at 400 nm, 5-aminosalicylic acid, giving a brown soluble product that absorbs especially at 450 nm, dicarboxidine dihydrochloride, which gives a brown soluble product absorbing especially at 440 nm, and o-phenylene diamine, giving an orange soluble product absorbing light particularly strongly at 492 nm as well as tetramethylbenzidine, which gives a blue insoluble product.

Further examples of suitable chromogenic co-reactants are 3-methyl-2-benzothiazolinone hydrazone/3-dimethylamino benzoic acid, giving a purple soluble product absorbing especially at 590 nm, diaminobenzidine, giving a brown insoluble product, and 4-chloro-1-naphthol, giving a blue insoluble product.

Coloured insoluble products can be especially usefully applied to qualitative detection tests, for example.

A fluorescent result can be achieved for example by using 3-(4-hydroxyphenyl) propionic acid as the co-reactant.

Alternatively, a luminescent coreactant can be employed. For example, in an embodiment of the test system described herein wherein a peroxide is present, luminol can be used to provide a detectable luminescence by reaction with the peroxide and peroxidase enzyme label.

Among the luminescent materials that can be used as a label without requiring an enzyme label are N-methylated 9-acridine carboxyl groups introduced for example by reaction of the substance to be labelled with the corresponding acid halide, followed by N-methylation.

## Claims

1. A process for carrying out a label-linked specific binding assay, eg an immunoassay, comprising the steps of
   - providing in an aqueous reaction zone a member of a specific binding pair to participate in the assay, linked to a label which either is a first coreagent which is reactive with a second coreagent in a reaction to produce a detectable assay result, or is a generator of said first coreagent;
   - providing a dispersed quantity of said second coreagent, or of a generator of said second coreagent, occluded or entrapped within a discretely bounded zone during production of the bounded zone, the bounded zone comprising cross-linked polymer, a polymer membrane microcapsule or a liposome, adjacent to said aqueous reaction zone, which bounded zone also carries in immobilised form at the zone boundary a member of a specific binding pair;
   - providing in said aqueous reaction zone a consumer of one of said coreagents in said reaction, thereby to ensure the substantial absence of said coreagent in said aqueous reaction zone;
   - wherein said discretely bounded zone comprises a barrier sufficient to prevent entry of the consumer reagent from said aqueous reaction zone into the bounded zone, thereby to separate the consumer reagent from the dispersed quantity of second coreagent or generator thereof within said bounded zone; and
   - allowing the specific binding reactions to take place whereby the assay reaction results in the binding of a variable quantity, dependent on the quantity of analyte present in the assay system, if any, of said labelled member of said specific binding pair directly or indirectly to said immobilised specific binding material, so that said detectable assay result is produced by reaction of bound label or said first coreagent generated by said bound label with said second coreagent occluded or entrapped within, or generated within, said bounded zone.

2. A process according to claim 1, wherein the discretely-bounded zone contains an enzymic or nonenzymic generator of the second coreagent.

3. A process according to claim 2, wherein the label is an enzyme and the second coreagent is a substrate for the enzyme.

4. A process according to claim 3, wherein there is also provided in the assay system a further substrate for the enzyme label.

5. A process according to claim 4, wherein the enzyme is a peroxidase, the second coreagent is hydrogen peroxide and the further substrate is a chromogenic, fluorogenic or luminescent substrate for the peroxidase, eg 2,2'-azino-di(3-ethylbenzthiazolinesulfonic acid), tetramethylbenzidine, 3-(4-hydroxyphenyl) propionic acid or luminol.

6. A process according to claim 1, wherein the label is a substance which can undergo a luminescent reaction and the second coreagent is a coreactant of the label in that reaction.

7. A process according to claim 6, wherein the label is an N-methylated acridine 9-carboxyl group and the second coreagent is hydrogen peroxide.

8. A process according to any of claims 1 to 7, in which the second coreagent is diffusible, is supplied or generated in the bounded zone, and its effective absence from the aqueous reaction zone is assured by a consumer reagent in the aqueous reaction zone that effectively consumes the second coreagent diffused from the bounded zone, thereby to prevent it from undergoing appreciable or substantial reaction with the label in the aqueous reaction zone.

9. A process according to claim 8, in which there is used a biphasic or two-compartment system in which the diffusible second coreagent is supplied or provided by a generating system in the first phase or compartment and diffuses into the second phase or compartment where it is consumed by the consumer reagent.

10. A specific binding assay process according to claim 1 or 9, in which (1) a gel particle or body containing a reactant-generator system for the second coreagent (such as a mixture of glucose and glucose oxidase, to make hydrogen peroxide) has immobilised specific binding agent on its surface (eg immobilised antibody), and is surrounded by a reaction medium containing a consumer reagent to consume any reactant that may diffuse away from the gel (such as catalase to consume the hydrogen peroxide); in which (2) the enzyme of the labelled binding material is capable, together with other reagent(s) in the system, of forming a detectable product when it comes into reactive contact with the second coreagent (eg the enzyme is a peroxidase and a chromogenic peroxidase substrate is provided to provide a colour result when these reagents come into effective contact with hydrogen peroxide); and in which (3) the specific binding event of the assay results in a (variable) extent of binding (according to the quantity of analyte in the system, if any) on the part of the enzyme-labelled binding material, to the complementary binding agent immobilised at the surface of the gel, so that the enzyme component of any material which does bind in this way comes into effective contact with the reactant and thereby forms the said detectable product.

11. A process according to claim 1, wherein polymer gel material, eg in the form of beadlets, having glucose oxidase entrapped therein (in the presence of glucose and oxygen), is provided with immobilised antigen or antibody or hapten on its surface to form the bounded zone, thereby to generate hydrogen peroxide in the bounded zone, as the second coreagent, and wherein catalase or a peroxidase is provided in the aqueous reaction zone to consume hydrogen peroxide which has diffused there without giving rise to a detectable product.

12. A process according to claim 1, wherein liposomes having glucose oxidase (in the presence of glucose and oxygen) or a photochemically-activatable hydrogen-peroxide-generating system entrapped therein, are provided with immobilised antigen or antibody or hapten on their surfaces to form the bounded zone, thereby to generate hydrogen peroxide in the bounded zone as the second coreagent and wherein catalase or a peroxidase is provided in the aqueous reaction zone to consume hydrogen peroxide which has diffused there without giving rise to a detectable product.

13. A test kit for carrying out the specific binding assay, eg immunoassay of any of claims 1 to 12, the test kit comprising
   - a member of a specific binding pair that participates in the binding reaction of interest in the assay, linked to a label material which either is a first coreagent which is reactive with a second coreagent in a reaction to produce a detectable assay result, or is a generator of said first coreagent,
   - a bounded disperse reagent preparation, comprising cross-linked polymer particles, polymer membrane microcapsules or liposomes, which constitutes a discretely-bounded zone containing occluded or entrapped therein a dispersed quantity either of said second coreagent in said reaction, or of a generator of said second coreagent, said bounded disperse reagent preparation also carrying in immobilised form at the zone boundary a member of a specific binding pair, and
   - a consumer reagent capable of consuming one of said coreagents,
   - wherein the zone boundary provides a barrier sufficient to prevent entry of the consumer reagent into said discretely-bounded zone, thereby to separate the consumer reagent from the dispersed quantity of second coreagent or generator thereof in said zone,
   - wherein the bounded disperse reagent preparation and the labelled material and the consumer reagent are arranged so that they can be put into contact during performance of the assay, with the labelled material and the consumer reagent dispersed in an aqueous zone adjacent to the bounded disperse reagent preparation, whereby in use the consumer reagent ensures the substantial absence of one of said coreagents in said aqueous reaction zone, and the assay reaction results in the binding of a variable quantity of said labelled material directly or indirectly to Said immobilized binding material, so that said detectable assay result is produced by reaction of said coreagent in said bounded disperse reagent preparation with said label bound thereto or with coreagent generated by said label.

**14.** A test kit according to claim 13, wherein said bounded dispersed reagent preparation comprises a preparation of beads or capsules to provide the bounded zone, carrying immobilized specific binding material and containing a hydrogen peroxide generating system as coreagent-generating system, and wherein said first reagent preparation comprises specific binding material linked to a peroxidase enzyme or a peroxide-reactive chemiluminescent label material, the test kit also comprising catalase, (thereby in use to consume any hydrogen peroxide in the aqueous reaction zone outside the beads or capsules), and optionally also a chromogenic or fluorogenic or luminescent material to give a detectable result upon reaction with the label and the reactant.

## Revendications

**1.** Procédé pour effectuer un titrage à liaisons spécifique par marqueur, par exemple un immunotitrage, qui consiste :
- à établir dans une zone de réaction aqueuse un élément d'une paire de liaison spécifique pour participer au titrage, liée à un marqueur qui est soit un premier coréactif qui réagit avec un second coréactif dans une réaction permettant d'obtenir un résultat de titrage détectable, ou qui est un générateur dudit premier coréactif;
- à fournir une quantité dispersée dudit second coréactif ou d'un générateur dudit second coréactif, occluse ou emprisonnée dans une zone séparément délimitée au cours de la production de la zone délimitée, la zone délimitée comprenant un polymère réticulé, une microcapsule à membrane de polymère ou un liposome adjacent à ladite zone de réaction aqueuse, cette zone délimitée portant également sous une forme immobilisée à la limite de la zone un élément d'une paire de liaisons spécifiques;
- à établir dans ladite zone de réaction aqueuse un consommateur de l'un desdits coréactifs dans ladite réaction pour assurer ainsi l'absence pratique dudit coréactif dans ladite zone de réaction aqueuse ;
- procédé dans lequel ladite zone séparément délimitée comprend une barrière suffisante pour empêcher l'entrée du réactif du consommateur depuis ladite zone de réaction aqueuse dans la zone délimitée, pour ainsi séparer le réactif consommateur de la quantité dispersée du second coréactif ou de son générateur dans ladite zone délimitée ; et
- à permettre les réactions de liaisons spécifiques de telle sorte que la réaction de titrage résulte dans la liaison d'une quantité variable, qui dépend de la quantité de l'analyte présent dans le système éventuel de titrage, dudit élément marqué de ladite paire de liaison spécifique directement ou indirectement à ladite matière immobilisée de liaison spécifique , de telle sorte que le résultat dudit titrage détectable soit produit par réaction du marqueur lié ou dudit premier coréactif engendré par ledit marqueur lié alors que le second coréactif est occlus ou emprisonné dans ladite zone délimitée ou engendré dans celle-ci.

**2.** Procédé selon la revendication 1, dans lequel la zone séparément délimitée contient un générateur enzymatique ou non enzymatique du second coréactif.

**3.** Procédé selon la revendication 2, dans lequel le marqueur est une enzyme et le second coréactif est un substrat pour l'enzyme.

**4.** Procédé selon la revendication 3, dans lequel on prévoit également dans le système de titrage un autre substrat pour le marqueur d'enzyme.

**5.** Procédé selon la revendication 4, dans lequel l'enzyme est une peroxidase, le second coréactif est le peroxyde d'hydrogène et le substrat supplémentaire est un substrat chromogène, fluorogène ou luminescent pour la peroxidase, par exemple le 2,2'-azino-di(acide 3-éthyl-benzothiazolinesulfonique), la tétraméthylbenzidine, 1' acide 3-(4-hydroxyphényl)propionique ou le luminol.

**6.** Procédé selon la revendication 1, dans lequel le marqueur est une substance pouvant subir une réaction luminescente et le second coréactif est un coréactif du marqueur dans cette réaction.

**7.** Procédé selon la revenditation 6, dans lequel le marqueur est un groupe acridine-9-carboxyle N-méthylé et le second coréactif est le peroxyde d'hydrogène.

8. Procédé selon l'une quelconque des revendications 1 à 7, dans lequel le second coréactif est diffusable, est fourni ou engendré dans la seconde zone délimitée et son absence réelle de la zone de réaction aqueuse est assurée par un réactif consommateur dans la zone de réaction aqueuse qui consomme efficacement le second coréactif diffusé depuis la zone délimitée, pour ainsi l'empêcher de subir une réaction notable ou importante avec le marqueur dans la zone de réaction aqueuse.

9. Procédé selon la revenditation 8, dans lequel on utilise un système biphasé ou à deux compartiments dans lequel le second coréactif diffusable est introduit ou fourni par un système générateur dans la première phase ou compartiment et il est diffusé dans la seconde phase ou compartiment où il est consommé par le réactif consommateur.

10. Procédé de titrage à liaisons spécifiques selon la revendication 1 ou 9, dans lequel (1) une particule ou corps de gel contenant un système générateur de réactif pour le second coréactif (tel qu'un mélange de glucose et de glucose-oxidase pour préparer du peroxyde d'hydrogène) a immobilisé l'agent de liaisons spécifiques sur sa surface (par exemple un anticorps immobilisé), et est entouré d'un milieu de réaction contenant un réactif consommateur pour consommer tout réactif pouvant être diffusé depuis le gel (tel qu'une catalase pour consommer le peroxyde d'hydrogène) ; dans lequel (2) l'enzyme du matériau de liaison marqué est capable, ensemble avec le ou les autres réactifs dans le système, de former un produit détectable, quand elle vient en contact réactif avec le second coréactif (par exemple l'enzyme est une peroxidase et on prévoit un substrat de peroxidase chromogène pour fournir un résultat en couleur quand ces réactifs viennent en contact efficace avec le peroxyde d'hydrogène) ; et dans lequel (3) l'apparition spécifique de liaison d'un titrage a pour effet un degré variable de liaison (selon la quantité de l'éventuel analyte dans le système) de la part de la matière de liaison marquée par enzyme, vers l'agent de liaison complémentaire immobilisé à la surface du gel, si bien que le composant enzyme d'un matériau quelconque qui n'est pas ainsi lié vient en contact efficace avec le réactif et forme ainsi ledit produit détectable.

11. Procédé selon la revendication 1, dans lequel le gel de polymère, par exemple sous forme de petites perles contenant une glucose-oxidase emprisonnée (en présence de glucose et d'oxygène) est munie d'un antigène ou anticorps ou haptène immobilisé à sa surface pour former la zone délimitée, pour ainsi engendrer du peroxyde d'hydrogène dans la zone délimitée, à titre de second coréactif, et dans lequel une catalase ou une peroxidase est prévue dans la zone de réaction aqueuse pour consommer le peroxyde d'hydrogène diffusé dans celle-ci sans créer de produit détectable.

12. Procédé selon la revendication 1, dans lequel des liposomes contenant la glucose-oxidase (en présence de glucose et d'oxygène) ou un système générateur de peroxyde d'hydrogène activable par voie photochimique qui est emprisonné, sont munis d'un antigène ou anticorps ou haptène immobilisé sur leurs surfaces pour former la zone délimitée, pour ainsi engendrer du peroxyde d'hydrogène dans la zone délimitée à titre de second coréactif et dans lequel la catalase ou une peroxidase est prévue dans la zone de réaction aqueuse pour consommer le peroxyde d'hydrogène qui a été diffusé sans donner naissance à un produit détectable.

13. Trousse de test pour effectuer le titrage à liaisons spécifiques, par exemple un immunotitrage selon l'une quelconque des revendications 1 à 12, trousse qui comprend :
   - un élément d'une paire de liaisons spécifiques qui participe dans la réaction de liaison pertinente dans le titrage, liée à un matériau marqueur qui est soit un premier coréactif pouvant réagir avec un second coréactif au cours de la réaction pour produire un résultat de titrage détectable, soit un générateur dudit premier coréactif ,
   - une préparation d'un réactif dispersé délimité, comprenant des particules d'un polymère réticulé, des microcapsules à membrane polymère ou des liposomes, constituant une zone séparément délimitée contenant une quantité dispersée occluse ou emprisonnée soit dudit second coréactif dans ladite réaction, soit d'un générateur dudit second coréactif, ladite préparation du réactif dispersé délimité portant aussi sous une forme immobilisée dans la zone frontière un élément d'une paire de liaisons spécifiques, et
   - un réactif consommateur capable de consommer l'un desdits coréactifs,
   - dans laquelle la délimitation de la zone fournit une barrière suffisante pour empêcher l'entrée du réactif consommateur dans ladite zone séparément délimitée, pour ainsi séparer le réactif consommateur de la quantité dispersée du second coréactif ou de son générateur dans ladite zone,

- dans laquelle la préparation réactive dispersée délimitée et le matériau marqueur ainsi que le réactif consommateur sont agencés de façon à pouvoir être mis en contact pendant le déroulement du titrage, le matériau marqué et le réactif consommateur étant dispersé dans la zone aqueuse adjacente à la préparation de réactif dispersé délimité , de sorte qu'en utilisation, le réactif consommateur assure l'absence pratique de l'un desdits coréactifs dans ladite zone de réaction aqueuse, et la réaction de titrage a pour effet la liaison d'une quantité variable de ladite matière marquée directement ou indirectement à ladite matière de liaison immobilisée, de sorte qu'on obtient le résultat dudit titrage détectable par réaction dudit coréactif dans ladite préparation du réactif dispersé délimité à laquelle est lié ledit marqueur ou le coréactif engendré par ledit marqueur.

14. Trousse de test selon la revendication 13, dans laquelle ladite préparation du réactif dispersé délimité comprend une composition de perles ou capsules pour définir la zone délimitée, portant une matière immobilisée de liaisons spécifiques et contenant un système générateur de peroxyde d'hydrogène à titre de système générateur de coréactif et dans laquelle ladite première préparation de réactif comprend une matière de liaison spécifique liée à une enzyme peroxidase ou une matière de marquage chimioluminescente réactive avec le peroxyde, la trousse de test comprenant également une catalase (servant à consommer le peroxyde d'hydrogène éventuel dans la zone de réaction aqueuse en dehors des perles ou capsules) et, facultativement, également une matière chromogène ou fluorogène ou luminescente pour donner un résultat détectable lors de la réaction avec le marqueur et le réactif.

## Patentansprüche

1. Verfahren zur Durchführung eines marker-gebundenen spezifischen Bindungstestes, z.B. eines Immunoassays, das die Stufen umfaßt:
   - Bereitstellung in einer wäßrigen Reaktionszone eines Mitgliedes eines spezifischen Bindungspaares zur Teilnahme am Test, gebunden an einen Marker, der entweder ein erstes Koreagenz, das mit einem zweiten Koreagenz in einer Reaktion zur Bildung eines feststellbaren Testergebnisses reaktionsfähig ist, oder ein Bildner des ersten Koreagenzes ist;
   - Bereitstellung einer dispergierten Menge des zweiten Koreagenzes oder eines Bildners des zweiten Koreagenzes, eingeschlossen oder eingefangen in einer diskret begrenzten Zone während der Bildung der begrenzten Zone, wobei die begrenzte Zone ein vernetztes Polymer, eine Polymermembran-Mikrokapsel oder ein Liposom, angrenzend an die wäßrige Reaktionszone, umfaßt und die begrenzte Zone ferner an der Zonenbegrenzung in immobilisierter Form ein Mitglied eines spezifischen Bindungspaares trägt;
   - Bereitstellung in der wäßrigen Reaktionszone eines Consumer-Reagenzes eines der Koreagenzien in dieser Reaktion, wodurch sichergestellt wird, daß dieses Koreagenz in der wäßrigen Reaktionszone praktisch nicht vorliegt;
   - worin die diskret begrenzte Zone eine ausreichende Schranke umfaßt, um einen Eintritt des Consumer-Reagenzes aus der wäßrigen Reaktionszone in die begrenzte Zone zu verhindern, wodurch das Consumer-Reagenz von der dispergierten Menge des zweiten Koreagenzes oder des Bildners desselben innerhalb der begrenzten Zone getrennt wird, und
   - Stattfindenlassen der spezifischen Bindungsreaktionen, wodurch die Testreaktion zur direkten oder indirekten Bindung einer variablen Menge, die von der im Testsystem (gegebenenfalls) vorliegenden Menge eines Analyten abhängt, des markierten Mitglieds des spezifischen Bindungspaares an das immobilisierte spezifische Bindungsmaterial führt, so daß das feststellbare Testergebnis durch Reaktion des gebundenen Markers oder des ersten Koreagenzes, das durch den gebundenen Marker mit dem zweiten Koreagenz gebildet wird, das in der begrenzten Zone eingeschlossen oder eingefangen ist oder innerhalb derselben gebildet wird, erzielt wird.

2. Verfahren gemäß Anspruch 1, bei welchem die diskret begrenzte Zone einen enzymatischen oder nicht-enzymatischen Bildner des zweiten Koreagenzes enthält.

3. Verfahren gemäß Anspruch 1, bei welchem der Marker ein Enzym ist und das zweite Koreagenz ein Substrat für das Enzym ist.

4. Verfahren gemäß Anspruch 3, bei welchem im Testsystem noch ein weiteres Substrat für den Enzymmarker bereitgestellt wird.

5. Verfahren gemäß Anspruch 4, bei welchem das Enzym eine Peroxidase ist, das zweite Koreagenz Wasserstoffperoxid ist und das weitere Substrat ein chromogenes, fluorogenes oder leuchtendes Substrat für die Peroxidase, z.B. 2,2'-Azino-di-(3-ethylbenzthiazolin-sulfonsäure), Tetramethylbenzidin, 3-(4-Hydroxphenyl)-propionsäure oder Luminol, ist.

6. Verfahren gemäß Anspruch 1, bei welchem der Marker eine Substanz ist, die sich einer Leuchtreaktion unterziehen kann, und das zweite Koreagenz ein Koreaktionsteilnehmer der Markers in dieser Reaktion ist.

7. Verfahren gemäß Anspruch 6, bei welchem der Marker eine N-methyliertes Acridin-9-carboxylgruppe ist und das zweite Koreagenz Wasserstoffperoxid ist.

8. Verfahren gemäß irgendeinem der Ansprüche 1 bis 7, bei welchem das zweite Koreagenz diffundierbar ist, in der begrenzten Zone vorliegt oder gebildet wird und seine effektives Fehlen in der wäßrigen Reaktionszone durch ein Consumer-Reagenz in der wäßrigen Reaktionszone sichergestellt wird, das das zweite, aus der begrenzten Zone diffundierte Koreagenz wirksam verbraucht, um so zu verhindern, daß es einer merklichen oder wesentlichen Reaktion mit dem Marker in der wäßrigen Reaktionszone unterliegt.

9. Verfahren gemäß Anspruch 8, bei welchem ein biphasiges oder Zwei-Kammer-System verwendet wird, in welchem das diffundierbare zweite Koreagenz durch ein System seiner Bildung in der ersten Phase oder Kammer geliefert oder bereitgestellt wird und in die zweite Phase oder Kammer diffundiert, wo es ·durch das Consumer-Reagenz verbraucht wird.

10. Spezifisches Bindungstestverfahren gemäß Anspruch 1 oder 9, bei welchem (1) ein Gelpartikel oder -körper, der ein einen Reaktionsteilnehmer bildendes System für das zweite Koreagenz enthält (z.B. eine Mischung aus Glucose und Glucose-Oxidase zur Bildung von Wasserstoffperoxid), auf seiner Oberfläche ein immobilisiertes spezifisches Bindungsmittel (z.B. einen immobilisierten Antikörper) aufweist und von einem Reaktionsmedium umgeben ist, das ein Consumer-Reagenz zum Verbrauchen irgendeines Reaktionsteilnehmers enthält, der aus dem Gel herausdiffundieren könnte (z.B. Catalase zum Verbrauchen des Wasserstoffperoxids), bei welchem (2) das Enzym des markierten Bindungsmaterials zusammen mit dem oder den anderen Reagenz(ien) im System fähig ist, ein feststellbares Produkt zu bilden, wenn es mit dem zweiten Koreagenz in reaktiven Kontakt kommt (ist das Enzym z.B. eine Peroxidase, so wird ein chromogenes Peroxidase-Substrat unter Bildung eines Farbergebnisses bereitgestellt, wenn diese Reagenzien mit Wasserstoffperoxid in wirksamen Kontakt kommen) und bei welchem (3) das spezifische Bindungsereignis des Testes zu einem (variablen) Ausmaß einer Bindung (entsprechend der Menge des gegebenenfalls im System vorliegenden Analyten) seitens des enzymmarkierten Bindungsmaterials an das auf der Oberfläche des Gels immobilisierte, komplementäre Bindungsmittel führt, so daß die Enzymkomponente irgendeines Materials, das in dieser Weise bindet, in wirksamen Kontakt mit dem Reaktionsteilnehmer kommt und dadurch das feststellbare Produkt bildet.

11. Verfahren gemäß Anspruch 1, bei welchem ein Polymergel-Material, z.B. in Form von Perlchen mit darin eingschlossener Glucose-Oxidase (in Gegenwart von Glucose und Sauerstoff) mit immobilisiertem Antigen oder Antikörper oder Hapten auf seiner Oberfläche zur Bildung einer begrenzten Zone versehen wird, um so Wasserstoffperoxid in der begrenzten Zone als zweites Koreagenz zu bilden, und bei welchem Catalase oder eine Peroxidase in der wäßrigen Reaktionszone bereitgestellt wird, um Wasserstoffperoxid zu verbrauchen, das dorthin diffundiert ist, ohne ein feststellbares Produkt zu ergeben.

12. Verfahren gemäß Anspruch 1, bei welchem Liposomen, mit Glucose-Oxidase (in Gegenwart von Glucose und Sauerstoff) oder einem photochemisch aktivierbaren, Wasserstoffperoxid bildenden System darin eingeschlossen, mit immobilisiertem Antigen oder Antikörper oder Hapten auf ihren Oberflächen zur Bildung der begrenzten Zone versehen werden, um dadurch Wasserstoffperoxid in der begrenzten Zone als das zweite Koregenz zu bilden, und bei welchem Catalase oder eine Peroxidase in der wäßrigen Reaktionszone bereitgestellt wird, um Wasserstoffperoxid zu verbrauchen, das dorthin

17

diffundiert ist, ohne ein feststellberes Produkt zu ergeben.

13. Ein Test-Kit zur Durchführung des spezifischen Bindungstests, z.B. eines Immunoassays, gemäß irgendeinem der Ansprüche 1 bis 12, das umfaßt:

- ein Mitglied eines spezifischen Bindungspaares, das an der interessierenden Bindungsreaktion im Test teilnimmt, gebunden an ein Markermaterial, das entweder ein erstes Koreagenz, das mit einem zweiten Koreagenz in einer Reaktion zur Bildung eines feststellbaren Testergebnisses reaktionsfähig ist, oder ein Bildner des ersten Koreagenzes ist,
- ein begrenztes disperses Reagenz-Präparat, umfassend vernetzte Polymerteilchen, Polymermembran-Mikrokapseln oder Liposomen, das eine diskret begrenzte Zone darstellt, die eine dispergierte Menge entweder des zweiten Koreagenzes in der Reaktion oder eines Bildners des zweiten Koreagenzes darin eingeschlossen oder eingefangen enthält, wobei das begrenzte disperse Reagenz-Präparat ferner an der Zonenbegrenzung in immobilisierter Form ein Mitglied eines spezifischen Bindungspaares trägt, und
- ein Verbraucher-Reagenz, das eines der Koreagenzien verbrauchen kann,
- bei welchem die Zonenbegrenzung eine ausreichende Schranke liefert, um den Eintritt des Consumer-Reagenzes in die diskret begrenzte Zone zu verhindern und um so das Consumer-Reagenz von der dispergierten Menge des zweiten Koreagenzes oder dessen Bildners in dieser Zone zu trennen,
- bei welchem das begrenzte disperse Reagenz-Präparat und das markierte Material sowie das Consumer-Reagenz so angeordnet sind, daß sie während der Durchführung des Testes mit dem markierten Material und dem Consumer Reagenz, das in einer wäßrigen, an das begrenzte disperse Reagenz-Präparat angrenzenden Zone dispergiert ist, in Kontakt gebracht werden können, wodurch bei der Verwendung das Consumer-Reagenz die praktische Abwesenheit einer dieser Koreagenzien in der wäßrigen Reaktionszone sicherstellt und die Testreaktion zur direkten oder indirekten Bindung einer variablen Menge des markierten Materials an das immobilisierte Bindungsmaterial führt, so daß das feststellbare Testergebnis durch die Reaktion des Koreagenzes im begrenzten dispersen Reagenz-Präparat mit dem darin gebundenen Marker oder mit dem durch diesen Marker gebildeten Koreagenz herbeigeführt wird.

14. Test-Kit gemäß Anspruch 13, in welchem das begrenzte dispergierte Reagenz-Präparat ein Präparat von Perlen oder Kapseln zur Bildung der begrenzten Zone umfaßt, die immobilisiertes spezifisches Bindungsmaterial trägt und ein Wasserstoffperoxid bildendes System als das das Koreagenz bildende System enthält, und in welchem das erste Reagenz-Präparat spezifisches Bindungsmaterial umfaßt, das ein Peroxidase-Enzym oder ein mit Peroxid reaktionsfähiges, chemisch leuchtendes Markermaterial umfaßt, wobei das Test-Kit ferner Catalase (um bei der Verwendung jegliches Wasserstoffperoxid in der wäßrigen Reaktionszone außerhalb der Perlen oder Kapseln zu verbrauchen) und wahlweise noch ein chromogenes oder fluorogenes oder leuchtendes Material umfaßt, um nach Reaktion mit dem Marker und dem Reaktionsteilnehmer ein feststellbares Ergebnis zu liefern.